# EUROPEAN PATENT SPECIFICATION

(11) **EP 1 789 099 B1**
(45) Date of publication and mention of the grant of the patent: **17.02.2010**
(21) Application number: 05761349.9
(22) Date of filing: 19.07.2005
(51) Int. Cl.: A61L 9/05, E03D 9/03, C11D 17/00

(54) **IMPROVED DISPENSING DEVICE**
VERBESSERTE ABGABEVORRICHTUNG
DISPOSITIF DE DISTRIBUTION AMELIORÉ

(30) Priority: 04.08.2004 GB 0417361
(43) Date of publication of application: 30.05.2007
(73) Proprietor: Reckitt Benckiser Inc., Parsippany, New Jersey 07054 (US)
(72) Inventor: NAPOLITANO, Lisa, Ann, New Jersey, NJ 07621 (US); NGUYEN, Lamson, Somerset, NJ 08873 (US)
(74) Representative: Bowers, Craig Malcolm
(86) International application number: PCT/GB2005/002843
(87) International publication number: WO 2006/013322

(56) References cited:
- EP-A- 0 960 984
- EP-A- 1 469 132
- WO-A-03/042462
- WO-A-2004/001145
- US-A- 4 709 424

## Description

The present invention is related to a device which is useful in dispensing a treatment composition (e.g, cleaning and/or sanitizing and/or coloring composition) to a sanitary appliance, e.g., a toilet bowl, while simultaneously dispensing a fragrance or perfume to the ambient environment outside of the sanitary appliance.

Since the advent of sanitary appliances, there has been a continuing need in the art to provide effective ways to maintain these appliances in a satisfactory condition between uses. The art is replete with devices which are intended to be used as "in the bowl" (or ITB) or "in the cistern" (or ITC) in order to provide a coloring and/or cleaning and/or fragrancing and/or sanitizing effect to such sanitary devices, particularly toilet bowls. While many of these devices are known and widely used they are not without drawbacks. One common technical problem is to ensure the effective delivery of a treatment agent, especially a coloring agent and/or a cleaning and/or a sanitizing agent to the interior of a toilet, while at the same time providing a fragrancing effect in the proximity of the toilet. One common approach known to the art is to provide a device which is suspended from the rim of the toilet bowl and which is placed at or near the interior sidewall of the toilet bowl. Such a device is designed to typically dispense a treatment composition to the interior of a toilet typically when contacted with flushing water, or alternately, it dispenses a fragrancing composition to the toilet bowl which is intended to counteract or mask malodors. Certain known arts devices can provide these effects simultaneously, however they are frequently limited in their application due to the requirements of chemical compatibility between several chemical compositions which are needed in order to provide these different treatment effects. Such chemical compatibility requirements curtail the range of possible treatment compositions which may be simultaneously used.

From US 4709424, EP0960984 and WO 2004/001145 it is known to provide a device with two compartments for a treatment composition.

Thus, while certain known-art dispensing devices provide beneficial treatment effects, there is nonetheless a real and continuing need in the art to provide improved devices which can simultaneously provide to a sanitary appliance, e.g., a toilet bowl, while simultaneously dispensing a fragrance or perfume to the ambient environment outside of the sanitary appliance without the necessity of ensuring chemical compatibility between the fragrancing composition and the one or more treatment compositions present in the device.

The present invention is defined in claim 1 and, various aspects thereof are defined in claims 2-11. The device can be used either as an ITC type device, or an ITB type device for a toilet bowl, but is preferably used as an ITB type device.

In a further aspect of the invention, there is provided a dispensing device according to any prior described inventive aspect, which comprises three or more dispenser bodies, at least two dispenser bodies of which depend from a bridge element or which depend from a hinge.

In a further aspect of the present invention there is provided a process for the delivery of a plurality of treatment compositions to the interior of a sanitary appliance, particularly a toilet bowl, which process contemplates providing a dispensing device as described herein, installing the device within or upon at least a portion of a sanitary appliance, particularly a toilet bowl, such that more than one chemical composition contained within the dispensing device contacts water and forms different treatment compositions used for treating the sanitary appliance, and such that these different treatment compositions egress from the dispensing device at different rates.

These and still further aspects of the invention will become more apparent from the following detailed description of the invention, and accompanying drawings.
Figure 1 illustrates a perspective frontal view of a dispensing device.
Figure 2 illustrates a perspective rear view of the device of Fig. 1.
Figure 3 illustrates the mounting of the device of Figures 1 and 2 with respect to a toilet bowl.
Figure 4 illustrates a perspective frontal view of a dispensing device.
Figure 5 illustrates a partial perspective rear view of illustrating the interior of the device of Fig. 4.
Figure 6 illustrates a perspective frontal view of a dispensing device.
Figure 7 illustrates a perspective frontal view of a dispensing device.
Figure 8 illustrates a perspective frontal view of a further dispensing device.
Figure 9 illustrates a top planar view of the embodiment disclosed on Figure 7, mounted on a toilet bowl.
Figure 10 illustrates a top planar view of an embodiment.
Figure 11 illustrates a top planar view of an embodiment.
Figures 12A and 12B illustrates embodiments of a hanger.
Figure 13 illustrates a perspective frontal view of a dispensing device. The hanger element has been omitted from this diagram for clarity.
Figure 14 illustrates a bottom planar view of the embodiment disclosed on Figure 13.
Figure 15 illustrates a rear planar view of the embodiment disclosed on Figure 13.
Figure 16 illustrates an exploded view of the embodiment disclosed on Figure 13.
Figure 17 illustrates a perspective frontal view of a dispensing device. The hanger element has been omitted from this diagram for clarity.
Figure 18 illustrates a top planar view of the embodiment disclosed on Figure 17.
Figure 19 illustrates a bottom planar view of the embodiment disclosed on Figure 17.

The device according to the invention is used to simultaneously deliver a treatment composition from at least a first dispenser body to a surface of a sanitary appliance, which treatment composition contains one or more active chemical agents e.g., coloring agents, cleaning agents, disinfecting agents, anti-lime scale agents, or is a mixture of two or more active chemical agents, while simultaneously providing a fragrancing effect from the device to the ambient environment of the sanitary appliance. A treatment composition is formed by the flow of water passing through the device coming into contact with a chemical composition contained within the first dispenser body, or the second dispenser body or contained within both dispenser bodies.

Both the chemical composition, as well as the fragrance composition may be in provided to the device in any physical form, e.g., in a liquid, gel or solid form. Conveniently however, the chemical composition is in a gel form or is in a solid form, such as in the form of dissolvable block or dissolvable pellets or particles which provides for the long term release of an active agent during sequential contacts with water entering and exiting the first housing of the device. Conveniently and most preferably the fragrancing composition, when present, is provided in a gel form.

The chemical composition may include any known art cleaning agents or cleaning constituents known to those of ordinary skill in the relevant art, and without limitation include one or more detersive surfactants selected from anionic, cationic, nonionic as well as amphoteric or zwitterionic surfactants. Certain detersive surfactants may also provide a dual role in providing detergency as well as a disinfecting effect, viz, certain cationic surfactants, which are described hereinafter as a disinfecting agent. These one or more cleaning agents or cleaning constituents may be used with or without other constituents being present in the chemical compositions of the invention.

By way of non-limiting example, useful anionic surfactants include the water-soluble salts, particularly the alkali metal, ammonium and alkylolammonium (e.g., monoethanolammonium or triethanolammonium) salts, of organic sulfuric reaction products having in their molecular structure an alkyl group containing from about 10 to about 20 carbon atoms and a sulfonic acid or sulfuric acid ester group. (Included in the term "alkyl" is the alkyl portion of aryl groups.) Examples of this group of synthetic surfactants are the alkyl sulfates, especially those obtained by sulfating the higher alcohols (C₈-C₁₈ carbon atoms) such as those produced by reducing the glycerides of tallow or coconut oil; and the alkylbenzene sulfonates in which the alkyl group contains from about 9 to about 15 carbon atoms, in straight chain or branched chain. Exemplary useful are linear straight chain alkylbenzene sulfonates in which the average number of carbon atoms in the alkyl group is from about 11 to 14.

Further exemplary useful anionic surfactants herein are the water soluble salts of: paraffin sulfonates containing from about 8 to about 24 (preferably about 12 to 18) carbon atoms; alkyl glyceryl ether sulfonates, especially those ethers of C₈₋₁₈ alcohols (e.g., those derived from tallow and coconut oil); alkyl phenol ethylene oxide ether sulfates containing from about 1 to about 4 units of ethylene oxide per molecule and from about 8 to about 12 carbon atoms in the alkyl group; and alkyl ethylene oxide ether sulfates containing about 1 to about 4 units of ethylene oxide per molecule and from about 10 to about 20 carbon atoms in the alkyl group.

Still further exemplary useful anionic surfactants herein include the water soluble salts of esters of α-sulfonated fatty acids containing from about 0 to 20 carbon atoms in the fatty acid group and from about 1 to 10 carbon atoms in the ester group; water soluble salts of 2-acyloxy-alkane-1-sulfonic acids containing from about 2 to 9 carbon atoms in the acyl group and from about 9 to about 23 carbon atoms in the alkane moiety; water-soluble salts of olefin sulfonates containing from about 12 to 24 carbon atoms; and β-alkyloxy alkane sulfonates containing from about 1 to 3 carbon atoms in the alkyl group and from about 8 to 20 carbon atoms in the alkane moiety.

A further class of anionic surfactants which may be used include carboxylates such as alkyl carboxylates which include those which may be represented by the general formula:

R-COO⁻M⁺

wherein R is a straight or branched hydrocarbon chain containing from about 9 to 21 carbon atoms, and M is a metal or ammonium ion; polyalkoxycarboxylates, representative of which are polyethoxycarboxylates which may be represented by the general formula:

R-[-OCH₂CH₂-]ₙ-CH₂COO⁻M⁺

wherein R is a straight chained or branched hydrocarbon chain which may include an aryl moiety, but is desirably a straight chained or branched hydrocarbon chain; and n is an integer value of from 1- 24.

Preferred anionic surfactants are those anionic surfactants typically used in toilet cleaning compositions. Examples include sulfonates, sulfates, carboxylates, phosphates, and mixtures of the above compounds. Suitable cations in this case are alkali metals such as, for example, sodium or potassium, or alkaline earth metals such as, for example, calcium or magnesium, and ammonium, substituted ammonium compounds, including mono-, di- or triethanolammonium cations and mixtures of the cations. The following types of anionic surfactants are of particular interest: alkyl ester sulfonates, alkylsulfates, alkyl ether sulfates, alkylaryl sulfates and sulfonates, and secondary alkanesulfonates, alkenyl sulfonates. Examples of suitable anionic surfactants include alpha olefin sulfonates, dodecylbenzene sulfonates, lauryl ether sulfates, lauryl monethanol amides.

Exemplary nonionic surfactants which may find use in the present invention include known art nonionic surfactant compounds. Practically any hydrophobic compound having a carboxy, hydroxy, amido, or amino group with a free hydrogen attached to the nitrogen can be condensed with ethylene oxide or with the polyhydration product thereof, polyethylene glycol, to form a water soluble nonionic surfactant compound. Further, the length of the polyethylenoxy hydrophobic and hydrophilic elements may various. Exemplary nonionic compounds include the polyoxyethylene ethers of alkyl aromatic hydroxy compounds, e.g., alkylated polyoxyethylene phenols, polyoxyethylene ethers of long chain aliphatic alcohols, the polyoxyethylene ethers of hydrophobic propylene oxide polymers, and the higher alkyl amine oxides.

A particularly useful class of nonionic surfactants include alkoxy block copolymers which include nonionic surfactants in which the major portion of the molecule is made up of block polymeric C₂-C₄ alkylene oxides. Such nonionic surfactants, while preferably built up from an alkylene oxide chain starting group, and can have as a starting nucleus almost any active hydrogen containing group including, without limitation, amides, phenols, thiols and secondary alcohols.

One group of such useful nonionic surfactants containing the characteristic alkylene oxide blocks are those which may be generally represented by the formula (A):

HO-(EO)ₓ(PO)_{y}(EO)_{z}-H (A)

where EO represents ethylene oxide,
PO represents propylene oxide,
y equals at least 15,
(EO)_{x+z} equals 20 to 80% of the total weight of said compounds, and,
the total molecular weight is preferably in the range of about 2000 to 15,000.

Another group of nonionic surfactants appropriate for use in the new compositions can be represented by the formula (B):

R-(EO,PO)ₐ(EO,PO)_{b}-H (B)

wherein R is an alkyl, aryl or aralkyl group, where the R group contains 1 to 20 carbon atoms, the weight percent of EO is within the range of 0 to 45% in one of the blocks a, b, and within the range of 60 to 100% in the other of the blocks a, b, and the total number of moles of combined EO and PO is in the range of 6 to 125 moles, with 1 to 50 moles in the PO rich block and 5 to 100 moles in the EO rich block.

Furtlier nonionic surfactants which in general are encompassed by Formula B include butoxy derivatives of propylene oxide/ethylene oxide block polymers having molecular weights within the range of about 2000-5000.
Still further useful nonionic surfactants containing polymeric butoxy (BO) groups can be represented by formula (C) as follows:

RO-(BO)ₙ(EO)ₓ-H (C)

wherein R is an alkyl group containing 1 to 20 carbon atoms,
n is about 5-15 and *x* is about 5-15.

Also useful as the nonionic block copolymer surfactants, which also include polymeric butoxy groups, are those which may be represented by the following formula (D) as follows :

HO-(EO)ₓ(BO)ₙ(EO)_{y}-H (D)

wherein n is about 5-15, preferably about 15,
x is about 5-15, preferably about 15, and
y is about 5-15, preferably about 15.

Still further useful nonionic block copolymer surfactants include ethoxylated derivatives of propoxylated ethylene diamine, which may be represented by the following formula: 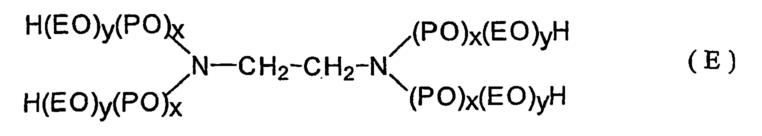 where (EO) represents ethoxy,
(PO) represents propoxy,
the amount of (PO)ₓ is such as to provide a molecular weight prior to ethoxylation of about 300 to 7500, and the amount of (EO)_{y} is such as to provide about 20% to 90% of the total weight of said compound.

Further exemplary useful nonionic surfactants which may be used in the present invention include certain alkanolamides including monoethanolamides and diethanolamides, particularly fatty monoalkanolamides and fatty dialkanolamides. Commercially available monoethanol amides and diethanol amides include those marketed under the trade names Alakamide® and Cyclomide® by Rhône-Poulenc Co., (Cranbury, NJ).

Preferred nonionic surfactants which may be used are those selected from primary and secondary alcohol ethoxylates and alkoxy block copolymers based on ethylene oxide, propylene oxide, and/or butylene oxide and mixtures thereof. For the alcohol ethoxylates, the alkyl chain of the aliphatic alcohols can be linear or branched, primary or secondary, and generally contains from about 8 to about 22 carbon atoms. The alkyl chain can be saturated or unsaturated. The alcohol ethoxylates can have a narrow ("narrow range ethoxylates") or a broad ("broad range ethoxylates") homolog distribution of the ethylene oxide. Examples of commercially available nonionic surfactants of this type are available under the tradenames Tergitol®, Genapol®, and Neodol®. Preferably, the alcohol ethoxylates are mixed C9/11 or C11/15 alcohol ethoxylates, condensed with an average of from 6 to 15 moles, preferably from 6 to 12 moles, and most preferably from 6 to 9 moles of ethylene oxide per mole of alcohol. Preferably the ethoxylated nonionic surfactant so derived has a narrow ethoxylate distribution relative to the average.

Further particularly preferred nonionic surfactants which may be used are nonionic surfactants based on block copolymers represented by formula (A) specific examples of which include those materials presently commercially available under the tradename Pluronic® (ex. BASF). Of those of formula (A), block copolymers having an average molecular weight between 7000 to 11,000 are preferred. Examples of such components include Pluronic® 87, described as EO₆₁ PO₄₁.₅ EO₆₁, having an average molecular weight of about 7700 and Pluronic® 88, described as EO₉₈ PO_{41.5} EO₉₈, having an average molecular weight of about 10800.

Non-limiting examples of exemplary useful amphoteric surfactants include alkylbetaines, particularly those which may be represented by the following structural formula:

RN(CH₃)₂CH₂COO⁻

wherein R is a straight or branched hydrocarbon chain which may include an aryl moiety, but is preferably a straight hydrocarbon chain containing from about 6 to 30 carbon atoms. Further exemplary useful amphoteric surfactants include amidoalkylbetaines, such as amidopropylbetaines which may be represented by the following structural formula:

RCONHCH₂CH₂CH₂N⁺(CH₃)₂CH₂COO⁻

wherein R is a straight or branched hydrocarbon chain which may include an aryl moiety, but is preferably a straight hydrocarbon chain containing from about 6 to 30 carbon atoms.

The chemical compositions of the invention may include one or more sanitizing agents or sanitizing constituents which may be used with or without other constituents being present in the chemical compositions of the invention.

The sanitizing agent can be any sanitizing composition known to those of ordinary skill in the relevant art, and without limitation exemplary sanitzing compositions include materials containing alkyl halohydantoins, alkali metal haloisocyanurates, essential oils, non-quatemary ammonium based germicidal compounds as well as quaternary ammonium germicidal compounds. These one or more sanitizing agents may be used with or without other constituents being present in the chemical compositions of the invention.

By way of non-limiting example, exemplary useful halohydantoins which may be used include those which may be represented by the general structure: 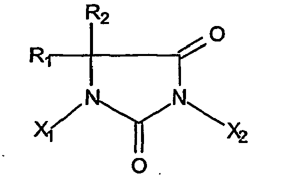 wherein:
X₁ and X₂ are independently hydrogen, chlorine or bromine; and,
R₁ and R₂ are independently alkyl groups having from 1 to 6 carbon atoms.

Examples ofhalohydantoins include, for example, N,N'-dichloro-dimethyl-hydantoin, N-bromo-N-chloro-dimethyl-hydantoin, N,N'-dibromo-dimethyl-hydantoin, 1,4-dichloro, 5,5-dialkyl substituted hydantoin, wherein each alkyl group independently has 1 to 6 carbon atoms, N-monohalogenated hydantoins such as chlorodimethylhydantoin (MCDMH) and N-bromo-dimethylhydantoin (MBDMH); dihalogenated hydantoins such as dichlorodimethylhydantoin (DCDMH), dibromodimethylhydantoin (DBDMH), and 1-bromo-3-chloro-5,5,-dimethylhydantoin (BCDMH); and halogenated methylethylhydantoins such as chloromethylethylhydantion (MCMEH), dichloromethylethylhydantoin (DCMEH), bromomethylethylhydantoin (MBMEH), dibromomethylethylhydantoin (DBMEH), and bromochloromethylethylhydantoin (BCMEH), and mixtures thereof. These materials are more fully discussed in United States Patent Nos. 4,560,766; 4,537,897; and 4,564,424.

Other germicidally effective agents useful as sanitizing agents include sodium dichloroisocyanurate (DCCNa) and sodium dibromoisocyanurate. Further examples of non-quaternary ammonium based sanitizing agents include pyrithiones, dimethyldimethylol hydantoin, methylchloroisothiazolinone/methylisothiazolinone sodium sulfite, sodium bisulfite, imidazolidinyl urea, diazolidinyl urea, benzyl alcohol, 2-bromo-2-nitropropane-1,3-diol, formalin (formaldehyde), iodopropenyl butylcarbamate, chloroacetamide, methanamine, methyldibromonitrile glutaronitrile, glutaraldehyde, 5-bromo-5-nitro-1,3-dioxane, phenethyl alcohol, o-phenylphenol/sodium o-phenylphenol, sodium hydroxymethylglycinate, polymethoxy bicyclic oxazolidine, dimethoxane, thimersal dichlorobenzyl alcohol, captan, chlorphenenesin, dichlorophene, chlorbutanol, glyceryl laurate, halogenated diphenyl ethers, phenolic compounds, mono- and poly-alkyl and aromatic halophenols, resorcinol and its derivatives, bisphenolic compounds, benzoic esters (parabens), halogenated carbanilides, 3-trifluoromethyl-4,4'-dichlorocarbanilide, and 3,3',4-trichlorocarbanilide. More preferably, the non-cationic antimicrobial agent is a mono- and poly-alkyl and aromatic halophenol selected from the group p-chlorophenol, methyl p-chlorophenol, ethyl p-chlorophenol, n-propyl p-chlorophenol, n-butyl p-chlorophenol, n-amyl p-chlorophenol, sec-amyl p-chlorophenol, n-hexyl p-chlorophenol, cyclohexyl p-chlorophenol, n-heptyl p-chlorophenol, n-octyl p-chlorophenol, o-chlorophenol, methyl o-chlorophenol, ethyl o-chlorophenol, n-propyl o-chlorophenol, n-butyl o-chlorophenol, n-amyl o-chlorophenol, tert-amyl o-chlorophenol, n-hexyl o-chlorophenol, n-heptyl o-chlorophenol, o-benzyl p-chlorophenol, o-benzyl-m-methyl p-chlorophenol, o-benzyl-m, m-dimethyl p-chlorophenol, o-phenylethyl p-chlorophenol, o-phenylethyl-m-methyl p-chlorophenol, 3-methyl p-chlorophenol, 3,5-dimethyl p-chlorophenol, 6-ethyl-3-methyl p-chlorophenol, 6-n-propyl-3-methyl p-chlorophenol, 6-iso-propyl-3-methyl p-chlorophenol, 2-ethyl-3,5-dimethyl p-chlorophenol, 6-sec-butyl-3-methyl p-chlorophenol, 2-iso-propyl-3,5-dimethyl p-chlorophenol, 6-diethylmethyl-3-methyl p-chlorophenol, 6-iso-propyl-2-ethyl-3-methyl p-chlorophenol, 2-sec-amyl-3,5-dimethyl p-chlorophenol 2-diethylmethyl-3,5-dimethyl p-chlorophenol, 6-sec-octyl-3-methyl p-chlorophenol, p-chloro-m-cresol, p-bromophenol, methyl p-bromophenol, ethyl p-bromophenol, n-propyl p-bromophenol, n-butyl p-bromophenol, n-amyl p-bromophenol, sec-amyl p-bromophenol, n-hexyl p-bromophenol, cyclohexyl p-bromophenol, o-bromophenol, tert-amyl o-bromophenol, n-hexyl o-bromophenol, n-propyl-m,m-dimethyl o-bromophenol, 2-phenyl phenol, 4-chloro-2-methyl phenol, 4-chloro-3-methyl phenol, 4-chloro-3,5-dimethyl phenol, 2,4-dichloro-3,5-dimethylphenol, 3,4,5,6-terabromo-2-methylphenol, 5-methyl-2-pentylphenol, 4-isopropyl-3-methylphenol, para-chloro-meta-xylenol, dichloro meta xylenol, chlorothymol, and 5-chloro-2-hydroxydiphenylinethane.

Quaternary ammonium based sanitizing agents include any cationic surfactant which is known or may be found to provide a broad antibacterial or sanitizing function. Any cationic surfactant which satisfies these requirements may be used and are considered to be within the scope of the present invention, and mixtures of two or more cationic surface active agents, viz., cationic surfactants may also be used. Cationic surfactants are well known, and useful cationic surfactants may be one or more of those described for example in McCutcheon's Functional Materials, Vol.2, 1998*;* Kirk-Othmer, Encyclopedia of Chemical Technology, 4th Ed., Vol. 23, pp. 481-541 (1997), the contents of which are herein incorporated by reference.

Exemplary cationic surfactant compositions useful in the practice of the instant invention are quaternary ammonium compounds and salts thereof, which may be characterized by the general structural formula: 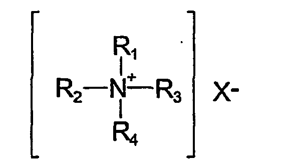 where at least one of R₁, R₂, R₃ and R₄ is a alkyl, aryl or alkylaryl substituent of from 6 to 26 carbon atoms, and the entire cation portion of the molecule has a molecular weight of at least about 165. The alkyl substituents may be long-chain alkyl, long-chain alkoxyaryl, long-chain alkylaryl, halogen-substituted long-chain alkylaryl, long-chain alkylphenoxyalkyl, arylalkyl, etc. The remaining substituents on the nitrogen atoms other than the abovementioned alkyl substituents are hydrocarbons usually containing no more than 12 carbon atoms. The substituents R₁, R₂, R₃ and R₄ may be straight-chained or may be branched, but are preferably straight-chained, and may include one or more amide, ether or ester linkages. The counterion X may be any salt-forming anion which permits water solubility of the quaternary ammonium complex. Such quaternary compounds are available under the BARDAC®, BARQUAT®, HYAMINE®, LONZABAC®, BTC®, and ONYXTDE® trademarks, which are more fully described in, for example, McCutcheon's Functional Materials (Vol. 2), North American Edition, 2001, and the respective product literature from the suppliers identified below. For example, BARDAC® 205M is described to be a liquid containing alkyl dimethyl benzyl ammonium chloride, octyl decyl dimethyl ammonium chloride; didecyl dimethyl ammonium chloride, and dioctyl dimethyl ammonium chloride (50% active) (also available as 80% active (BARDAC® 208M)); described generally in *McCutclzeon's* as a combination of alkyl dimethyl benzyl ammonium chloride and dialkyl dimethyl ammonium chloride); BARDAC® 2050 is described to be a combination of octyl decyl dimethyl ammonium chloride/didecyl dimethyl ammonium chloride, and dioctyl dimethyl ammonium chloride (50% active) (also available as 80% active (BARDAC® 2080)); BARDAC® 2250 is described to be didecyl dimethyl ammonium chloride (50% active); BARDAC® LF (or BARDAC® LF-80), described as being based on dioctyl dimethyl ammonium chloride (BARQUAT® MB-50, MX-50, OJ-50 (each 50% liquid) and MB-80 or MX-80 (each 80% liquid) are each described as an alkyl dimethyl benzyl ammonium chloride; BARDAC® 4250 and BARQUAT® 4250Z (each 50% active) or BARQUAT® 4280 and BARQUAT® 4280Z (each 80% active) are each described as alkyl dimethyl benzyl ammonium chloride/alkyl dimethyl ethyl benzyl ammonium chloride. Also, HYAMINE® 1622, described as diisobutyl phenoxy ethoxy ethyl dimethyl benzyl ammonium chloride (available either as 100% actives or as a 50% actives solution); HYAMINE® 3500 (50% actives), described as alkyl dimethyl benzyl ammonium chloride (also available as 80% active (HYAMINE® 3500-80); and HYAMINE® 23 89 described as being based on methyldodecylbenzyl ammonium chloride and/or methyldodecylxylene-bis-trimethyl ammonium chloride. (BARDAC®, BARQUAT® and HYAMINE® are presently commercially available from Lonza, Inc., Fairlawn, NJ). BTC® 50 NF (or BTC® 65 NF) is described to be alkyl dimethyl benzyl ammonium chloride (50% active); BTC® 99 is described as didecyl dimethyl ammonium chloride (50% active); BTC® 776 is described to be myristalkonium chloride (50% active); BTC® 818 is described as being octyl decyl dimethyl ammonium chloride, didecyl dimethyl ammonium chloride, and dioctyl dimethyl ammonium chloride (50% active) (available also as 80% active (BTC® 818-80%)); BTC® 824 and BTC® 835 are each described as being of alkyl dimethyl benzyl ammonium chloride (each 50% active); BTC® 885 is described as a combination of BTC® 835 and BTC® 818 (50% active) (available also as 80% active (BTC® 888)); BTC® 1010 is described as didecyl dimethyl ammonium chloride (50% active) (also available as 80% active (BTC® 1010-80)); BTC® 2125 (or BTC® 2125 M) is described as alkyl dimethyl benzyl ammonium chloride and alkyl dimethyl ethylbenzyl ammonium chloride (each 50% active) (also available as 80% active (BTC® 2125-80 or BTC® 2125 M)); BTC® 2565 is described as alkyl dimethyl benzyl ammonium chlorides (50% active) (also available as 80% active (BTC® 2568)); BTC® 8248 (or BTC® 8358) is described as alkyl dimethyl benzyl ammonium chloride (80% active) (also available as 90% active (BTC® 8249)); ONYXIDE® 3300 is described as n-alkyl dimethyl benzyl ammonium saccharinate (95% active). (BTC® and ONYXIDE® are presently commercially available from Stepan Company, Northfield, IL).

The chemical compositions of the invention may also comprise a coloring agent which imparts a color to the water in which it comes into contact, and especially which imparts color to the water contained within the sanitary appliance. Where the sanitary appliance is a toilet, desirably the coloring agent imparts a color to the water contained within the cistern, or within the toilet bowl particularly following the flush cycle of a toilet, or may impart a color in both locations. Such coloring agents have great consumer appeal, and indeed any known art coloring agent may be provided in any effective amount in order to impart a coloring effect. Colorants, especially dyes, are preferred when formulated as dry powders to enable direct incorporation into the tablet or block, however, liquid colorants may be employed in conjunction with suitable carriers. When present, colorants are desirably present in an amount from about 0.1 to 15 percent of the total weight of the chemical composition.

While such coloring agents may be used as the sole chemical composition contained within the inventive device, such coloring agents typically combined with cleaning effective amounts of one or more surfactants which provide an effective cleaning benefit.

As noted previously, the chemical compositions of the invention may comprise an anti-limescale agent, which can be classified as a cleaning agent in that it provides a cleaning effect to treated lavatory device surfaces. The anti-limescale agent can virtually any known anti-limescale agent compositions known to those of ordinary skill in the relevant art. For example, compositions containing anionic and/or nonionic surfactants together with typical anti-limescale agents, for example, amidosulfonic acid, bisulfate salts, organic acids, organic phosphoric salts, alkali metal polyphosphates, and the like. Examples of anti-lime scale agent compositions can be found in, for example, United States Patent No. 5,759,974; United States Patent No. 4460490; and United States Patent No. 4578207. Further examples of anti-limescale agents include organic acids (for example, citric acid, lactic acid, adipic acid, oxalic acid and the like), organic phosphoric salts, alkali metal polyphosphates, sulfonic, and sulfamic acids and their salts, bisulfate salts, EDTA, phosphonates, and the like.

The chemical compositions may also include other known-art additives in effective amounts, such as solubility control agents, water-softening agents, , preservatives, flow aids, water-soluble fillers, corrosion inhibitors, and the like.

It will be appreciated by those of ordinary skill in the art that several of the components which are directed to provide a chemical composition can be blended into one chemical composition with the additional appreciation that potential blending of incompatible components will be avoided. For example, those of ordinary skill in the art will appreciate that certain anionic surfactants may have to be avoided as some may be incompatible with certain sanitizing agents and/or certain anti-lime scale agents mentioned herein. Those of ordinary skill in the art will appreciate that the compatibility of the anionic surfactant and the various sanitizing and anti-limescale agents can be easily determined and thus incompatibility can be avoided in the situations.

When the chemical compositions are formed into solid blocks, such blocks can consist entirely of one or more of the chemical compositions which provide an active treatment benefit as described above but such blocks may also contain amounts of one or more active agents together with one or more adjuvants such as lubricants, as well as inactive adjuvants such as fillers known to the art which may be included in art recognized amounts.

Preferably when the chemical composition is in a solid block form, the chemical composition according to the invention is made up into a block of from about 25 to about 75g, more preferably from about 25 to about 55g, and more preferably from about 30 to about 45g. Of course it is to be understood that less than the total amount of the solid block formed may be the chemical composition, with the remaining balance being one or more adjuvants.

The solid block can be made by conventional means. One method of making the block is to melt one or more of the components and then pouring the molten mass into the first chamber of the housing and allowing the mass to cool to room temperature (about 25°C). Another method is to place the components used to make the block into an appropriate extrusion device and extrude an appropriately sized mass that will fit into the first and/or second chamber of the housing. If the solid block is to be made by extrusion, then processing aids are often included as needed and are included as an adjuvant. The solid block may be formed of a single chemical composition, or may formed of two different chemical compositions which may be provided as separate regions of a solid block, such as a first layer of a solid block consisting of a first chemical composition, alongside a second layer of a the solid block consisting of a second chemical composition which is different than the first chemical compositions. Further layers of still further different chemical compositions may also be present. Such solid blocks formed having two or more discrete layers or regions of, respectively, two or more different chemical compositions may be referred to as composite blocks. Such composite block may be formed by any conventional technique, including, e.g. forming the two (or more) layers or regions of the composite block separately and thereafter assembling the final composite block, as well as coextrusion techniques as known to the art.

When formed as a solid block, such solid blocks may be monolithic or alternately may be in particulate form such as in the form of powders, prills, beads and the like which particulate forms may be conveniently used. Alternately the chemical compositions may be in the form of a gel, which gel may optionally include one or more further solids, e.g., one or more further chemical compositions useful to treat a lavatory appliance within or mixed with the gel.

As noted, the device according to provides a fragrancing effect to the ambient environment of the sanitary appliance, which effect is provided by the presence of a fragrance composition. The fragrance composition may be any composition which is known to the art to provide a perceptible fragrancing benefit, any may be based on naturally occurring materials such as one or more essential oils, or may be based on synthetically produced compounds as well. Examples of essential oils include pine oil, Anetlhole 20/21 natural, Aniseed oil china star, Aniseed oil globe brand, Balsam (Perui), Basil oil (India), Black pepper oil, Black pepper oleoresin 40/20, Bois de Rose (Brazil) FOB, Bomneol Flakes (China), Camphor oil, White, Camphor powder synthetic technical, Canaga oil (Java), Cardamom oil, Cassia oil (China), Cedarwood oil (China) BP, Cinnamon bark oil, Cinnamon leaf oil, Citronella oil, Clove bud oil, Clove leaf, Coriander (Russia), Counmarin 69°C. (China), Cyclamen Aldehyde, Diphenyl oxide, Ethyl vanilin, Eucalyptol, Eucalyptus oil, Eucalyptus citriodora, Fennel oil, Geranium oil, Ginger oil, Ginger oleoresin (India), White grapefruit oil, Guaiacwood oil, Gurjun balsam, Heliotropin, Isobornyl acetate, Isolongifolene, Juniper berry oil, L-methyl acetate, Lavender oil, Lemon oil, Lemongrass oil, Lime oil distilled, Litsea Cubeba oil, Longifolene, Menthol crystals, Methyl cedryl ketone, Methyl chavicol, Methyl salicylate, Musk ambrette, Musk ketone, Musk xylol, Nutmeg oil, Orange oil, Patchouli oil, Peppermint oil, Phenyl ethyl alcohol, Pimento berry oil, Pimento leaf oil, Rosalin, Sandalwood oil, Sandenol, Sage oil, Clary sage, Sassafras oil, Spearmint oil, Spike lavender, Tagetes, Tea tree oil, Vanilin, Vetyver oil (Java), and Wintergreen oil.

Many of these essential oils may also function as a fragrance agent, which fragrance agent which may be a substance or mixture of such substances including those which are naturally derived (i.e., obtained by extraction of flower, herb, blossom or plant), those which are artificially derived or produced (i.e., mixture of natural oils and/or oil constituents), and those which are synthetically produced substances (odiferous substances). Generally fragrance agents are complex mixtures or blends various organic compounds including, but not limited to, certain alcohols, aldehydes, ethers, alamatic compounds and varying amounts of essential oils such as from about 0 to about 85% by weight, usually from about 10 to about 70% by weight, the essential oils themselves being volatile odiferous compounds and also functioning to aid in the dissolution of the other components of the fragrance agent. In the present invention, the precise composition of the fragrance agent desirably emanates a pleasing fragrance, but the nature of the fragrance agent is not critical to the success of the invention. Indeed, is fully contemplated as being within the scope of the invention to include any other material which is useful in providing treatment of ambient air, such as a sanitizing agents such as one or more glycols or alcohols, or materials which are intended to counteract, neutralize, or mask odors in place of, or in conjunction with the fragrance composition of the present invention. Alternatively, it is also contemplated that all or part of the fragrance composition of the present invention is may be substituted by one or more materials which provide and effective insecticide repelling or insecticidal benefit; such would be particularly useful in climates or environments where insects present a nuisance or health hazard.

According to particularly preferred embodiments according to the first aspect of the invention, the fragrance composition is associated solely with the fragrance cavity of the inventive devices. A according to preferred mode of utilizing the inventive device, the device is positioned with respect to a toilet bowl, such that the fragrance cavity does not come into contact with water during the useful life of the device. This provides several simultaneous benefits including, the longevity of the fragrance composition, the improved delivery characteristic of the fragrance composition which does not become submerged or diluted with water associated with the sanitary appliance, as well as the fact that a much broader range of fragrance compositions (or other air treatment compositions as noted above) can be utilized as there is no concern regarding the compatibility of fragrance with the materials in the chemical composition contained within a body cavity of the device. Furthermore, the utilization of the fragrance composition in such manner provides a constant release of the fragrance composition to the ambient environment of the sanitary appliance even when the sanitary appliance is not being the used. In the case where a pleasant fragrance and/or an odor making composition is provided in the fragrance composition, a beneficial consumer perception of the use of the devices can be realized. Alternately, where a sanitizing agent and/or an insecticidal agent is utilized as all or part of the fragrance composition of the device, the continual benefits of continuous release of such agents may be provided to the ambient environment of the sanitary applicance.

Notwithstanding the foregoing, it is to be understood that a chemical composition used in the device may also comprise a fragrance composition or other air treatment composition as described above. Such however exemplifies a less preferred inventive embodiment for the reasons noted herein.

The form of the fragrance composition, when present, can take any form including, liquid, solid, or gel form. Preferably however, the fragrance composition is a gel system which is then deposited in the fragrance chamber of the device. The gel system can be formed by a variety of components known to those of ordinary skill in the art. For example, it can be formed from absorbents, starch based systems, modified celluloses, natural gums and other materials which can form a gel when the fragrance composition, aforementioned gel components, and water or hydrophilic solvents are mixed together. According to certain particularly advantageous embodiments of the invention the fragrance composition is a gel system as it is described in United States Patent No. 5,780,527.

Examples of chemical compositions which can be used with the present invention are shown in the following table below.

| Component | Ex. 1 | Ex. 2 | Ex. 3 | Ex. 4 | Ex. 5 |
|---|---|---|---|---|---|
| Dodecyl Benzene Sulfonate Na¹ | 25 | 10 | 40 | 35 | 35 |
| Alfa Olefine Sulfonate Na² | 25 | 10 | 5 | 32 | 32 |
| Lauryl monoethanolamide³ | 10 | 8 | 5 | 2 | 5 |
| Sodium Lauryl Ether Sulfate⁴ | 10 | - | - | 4.5 | 5 |
| Pluronic 68⁵ | 10 | - | - | 3 | - |
| Na Sulfate | 20 | - | - | 21.5 | 21 |
| Pluronic 87 or 88⁶ | - | 70 | 50 | - | - |
| Alcohol ethoxylate C₉-C₁₁ 6EO⁷ | - | 2 | - | - | - |
| Silica | - | - | - | 2 | 2 |

| | | | | | |
|---|---|---|---|---|---|
| ¹ Dodecyl Benzene Sulfonate Sodium (80-90% active) - anionic ² Alpha Olefin Sulfonate Sodium - anionic ³ Lauryl Monoethanolamide -- non-ionic ⁴ Sodium Lauryl Ether Sulfate (70% active) -- anionic ⁵ Polyoxyethylene (160) polyoxypropylene (30) glycol - non-ionic ⁶ Pluronic 87 E₆₁ P_{41.5} E₆₁ - Molecular Weight 7700 - HLB 24 - non-ionic Pluronic 88 E₉₈ P_{41.5} E₉₈ -- Molecular Weight 10800 - HLB 28-- non-ionic ⁷ Alcohol ethoxylate C₉-C₁₁ 6EO -- non-ionic | | | | | |

The above exemplary chemical compositions can be made into solid blocks either by melting the various components together and placing the melt into a housing which is used as the first dispenser of the inventive device, or by placing the components into a suitable extruder and extruding out a block having a desired shape and size, and thereafter providing it to the first dispenser of the inventive device.

Exemplary sanitizing compositions for use as chemical compositions in the present invention include compositions having the general compositions described as follows:

A hydantoin tablet containing 94 wt. % Dantochlor powder (about 86% 1,3-dichloro-5,5-dimethylhydantoin) and 6.0 wt. % of an inert binder, comprising a 5 wt. % solution of laponite can be made by extrusion (with a die diameter and shape suited to the proposed first chamber) at a temperature of from about 80 to 90°F and a pressure at the end of the extruder barrel ranging from about 50 to about 350 psi. An appropriately sized block can then be cut from the extrudate and allowed to cool to room temperature. Another example can use a 2 wt. % solution of laponite. According to other examples the 5 wt. % solution of laponite can be replaced with sodium stearate and water (respectively representing 5 wt. % and 4 wt. % of composition prior to drying; respectively representing 10 wt. % and 6 wt. % of the final composition prior to drying; and respectively representing 6 wt. % and 7.5 wt. % of the composition, prior to drying). Alternately there can be used a binder that contains a 2 wt. % laponite solution and sodium stearate (the laponite solution representing 3 wt. % of the composition and the sodium stearate representing 7.5 wt. % of the composition, prior to drying; a 5 wt. % laponite solution and sodium stearate (respectively representing 3 wt. % and 7.5 wt. % of the composition, prior to drying).

An example of a bleach containing composition suitable for use as a chemical composition in the devices of the present invention include compositions having the following exemplary constituents present in the general ranges as follows:

| Constituent | range (%w/w) |
|---|---|
| Alpha olefin sulfonate | 0- 35 |
| Sodium lauryl ether sulfate | 3.0-6.0 |
| Bleaching agent (e.g., DCCNa or Hydantoin) | 0.5 - 25 |
| Lauryl monoethanolamide | 2.0-5.0 |
| Dodecyl benzene sulfonate Na | 50-70 |
| Na sulfate anhydrous | 15-25 |
| Silica | 1.0-2.0 |

A non-limiting examples of a anti-lime scale agent containing composition useful as a chemical composition in the devices of the present invention include compositions described as follows:

| Description | Qty |
|---|---|
| Spary dried silica | 9.46 |
| Na sulfate | 10.81 |
| Na dodecylbenzenesulfonate (80%) | 74.05 |
| Na₄ HEDP | 1.62 |
| Alcohol C₁₃/C₁₅ | 1.08 |
| Dye | 2.97 |

Particularly preferred embodiments of cleaning blocks which are useful in the present inventive compositions include those which comprise:
10 - 35%wt., preferably 15-30%wt. of an alpha olefin sulfonate anionic surfactant;
10 - 35%wt., preferably 15-30%wt. of a linear monoethanolamide;
5 - 50 %wt., preferably 15-35%wt. of a linear dodecylbenzene sulfonate anionic surfactant;
5 - 50%wt., preferably 20-35%wt. of sodium sulfate
0.1-15 %wt., preferably 0.5-5%wt. of silica
0.1 - 25%wt., preferably 1-10%wt. sodium lauryl ether sulfate
optionally to 40%wt. further additive constituents, including but not limited to further surfactants, fillers, binders, fragrances, processing aids such as lubricants and tabletting aids, bleaches, sanitizing compositions and the like.

The particularly preferred compositions exhibit a long service life when mounted in the devices described herein, which service life is believed to be superior to many known art ITB lavatory cleaning block compositions.

Particularly preferred compositions exhibit a long service life, and additionally exhibit a high ratio of surface area to block volume, preferably wherein the ratio of block volume to block surface area is at least about 0.25, preferably at least about 0.27, more preferably is at least about 0.29, and still more preferably is at least bout 0.30. The long service life of such blocks notwithstanding the large surface area of the blocks exposed to flush water entering the device is surprising.

It is to be understood that the configuration of the device permits for the use of various combinations of chemical treatment compositions and fragrance compositions. This is due to the fact that the each of the first and second dispenser bodies are constructed such that the chemical composition contained with the body cavity is physically separated from the fragrance composition which is present in the fragrance cavity, thus there is no need to ensure the chemical compatibility of chemical composition and fragrance composition present. Similarly, the body cavity and the fragrance cavity of the first dispenser body are physically separated from the body cavity and fragrance cavity of the second dispenser body, which obviates concern between chemical compatibility of the chemical and fragrance compositions in the respective first and second dispenser bodies as well. Such permits for wide variability in the selection and usage of chemical compositions and fragrance compositions in the inventive devices.

According to the invention the at least one chemical composition contained in the first dispenser body cavity is different from the at least one chemical composition contained in the second dispenser body cavity.
(i) wherein the fragrance compositions of the first dispenser fragrance cavity and of the second fragrance body may be the same; or
(ii) wherein the fragrance compositions of the first dispenser fragrance cavity and of the second dispenser fragrance cavity may be different.

In the devices according to the present invention, it is to be understood that the form of the first dispenser body and of the second dispenser body is not critical to the successful operation of the invention. It is only required that each dispenser body be appropriately dimensioned or appropriately sized in order that they may contain, or have otherwise associated therewith their chemical compositions, and in embodiments according to the first aspect of the invention, as well as their fragrancing compositions, and that each dispenser body depend from a bridge element, which is desirably flexible which flexibility permits for adaptive placement of the device in a toilet bowl. According to certain preferred embodiments of the invention, both the first dispenser body and the second dispenser body include a fragrance cavity having at least one surface open to the ambient environment, and in intermediate wall separating the fragrance cavity from a body cavity which is covered by a plate or other cover element which facilitates in retaining the chemical compositions contained within the body cavity.

A further element of the device according to the invention is a hanger adapted for removably hanging the device upon a portion of a sanitary appliance. Preferably the hanger depends from device at or near its midpoint between the two ends of the device. The form of the hanger may take any shape form or configuration which is found satisfactory. Ideally, the hanger is generally in the form of a rigid, semi-rigid or flexible strip or hook which may be made of a single element, such as a single flexible element, or which may be made from a plurality of separate elements which are linked or joined together such as an assemblage of jointed articulated sections adapted to be hung upon a portion of a sanitary applicance. In use, according to preferred methods for utilizing the dispenser, the hanger is used to suspend the device within the flow path of water within the sanitary appliance, while simultaneously suspending the device such that the fragrance composition does not normally into contact with water, nor within the flow path of water within the sanitary device. According to the method of use, the device is utilized in conjunction with a toilet bowl such that, the hanger suspends the device beneath the rim of a toilet bowl so that the dispenser body is at least partially suspended within the flow path of flowing water, e.g., flush water, within the sanitary appliance such that, during the release of flush water, at least part of flush water enters the body cavity.

Preferably the dispenser body is flexible so that it may approximately conform to the configuration of the sanitary appliance. More preferably the dispenser body is deformable so that it may be bent or shaped to approximately conform to the configuration of the sanitary appliance particularly in beneath the rim of a toilet bowl. Such flexure allows for a close placement within the flow path of water within the sanitary device and due to the rather large ratio of the maximum overall length dimension "L" and a maximum width dimension "W" measured in a plane perpendicularly intersecting "L" the area to which the treatment composition is supplied is greater than with known art prior devices. The inventors have surprisingly found that notwithstanding the larger ratio of "L" to "W", chemical compositions may be formulated and used in the devices with excellent cleaning and/or sanitization results while at the same time providing a useful service life to device. The large exposed surface area of the chemical compositions, particularly in the form of blocks, provide for good surface contact with flush water and excellent dispensing of the treatment composition to the toilet bowl. This is particularly enhanced where the flush water swirls within the interior of the toilet bowl which thus dispenses the treatment composition more effectively to the surfaces contacted with flush water. Desirably the dispenser body is sufficiently flexible, preferably is sufficiently permanently deformable such that the angle of deflection of the midpoint of at least one end of the dispenser body, as measured between an original, linear configuration and its deformed configuration as measured from the midpoint between the two ends of the dispenser body is at least 5°, preferably is at least about 8°, and most preferably is at least about 10° or more.

According to certain particularly preferred aspects of the invention there is provided a dispensing device as described herein wherein the dispensing device has a maximum overall length dimension "L" and a maximum width dimension "W" measured in a plane perpendicularly intersecting "L" wherein the device has its maximum dimension and wherein the value of "L" to the value of "W" is at least 2 × W, preferably is at least 2.5 × W, yet more is at least 3 × W, still more preferably is at least 3.5 × W, especially preferably is at least is at least 4 × W, yet more preferably is at least 4.5 × W, still more preferably is at least 5 × W, and most preferably is at least 6.5 × W. However, the maximum overall length dimension "L" does not exceed 15 × W.

The various elements of the device according to the invention can be formed out of any of a variety of materials with synthetic polymers being preferred. Exemplary suitable synthetic polymers include polyethylene, polypropylene, and the like; the only criteria being that the selected synthetic polymers is not affected by the components of the treatment composition, or fragrance composition particularly when in a gel form or solid form .

The device according to the invention may also have a different geometry and appearance than the embodiments described in the Figures. For example the hanger may be a rigid material or which may be a flexible material, such as in the form of a flexible band or strap. Further, each of the first and second dispenser bodies may have a configuration other than specific embodiments depicted in the Figures and indeed the configuration of the first and second dispenser bodies need not be the same in any device but can be different from one another.

In the accompanying figures, like elements are indicated using the same numerals throughout the figures.

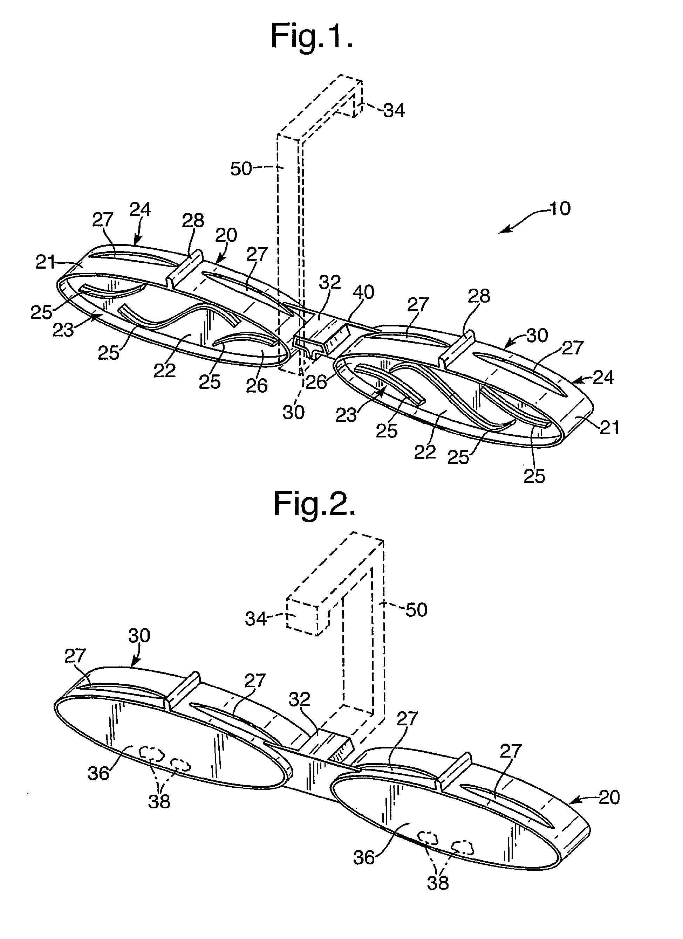

Figure 1 illustrates a perspective frontal view of a first preferred embodiment of a dispensing device 10 according to the first aspect of the invention which includes a first dispenser body 20 and a second dispenser body 30 each dependent from a bridge element 40. Shown in "phantom" is a representative hanger 50 which depends at one end from the bridge element 40; the use of phantom representation is used so as not to obscure the remaining elements in the figure. Each dispenser body includes within its interior a body sidewall 21 defining the margins of each dispenser body which is traversed by a divider wall 22 which is generally perpendicular to the body sidewall and within each of the respective first and second dispenser bodies. The divider wall physically separates each first and second dispenser bodies into two cavities, a frontwardly facing fragrance cavity 23 and a rearwardly facing body cavity 24. Depicted on the figure and within the fragrance cavity are several optional support walls 25 which extend frontwardly from the front face 26 of the divider wall; while the configuration, number and placement of such support walls are optional one or more supports walls are desirably provided in each fragrance cavity when the fragrance composition is based on a gel fragrance composition. The presence of such support walls aid in the retention of a gel fragrance composition during any shrinkage of the gel composition and inhibit its premature detachment from the device. Further visible on the figure are body cavity perforations 27 which define a passage through the body sidewall from the upper exterior sidewall and into the body cavity. A further optional element is a spacer wall 28 which extends upwardly from the exterior of the sidewall; although optional the presence of a spacer wall acts as a stand-off between the body cavity perforations and a part of the sanitary appliance and decreases the likelihood of blockage of either body cavity perforation.

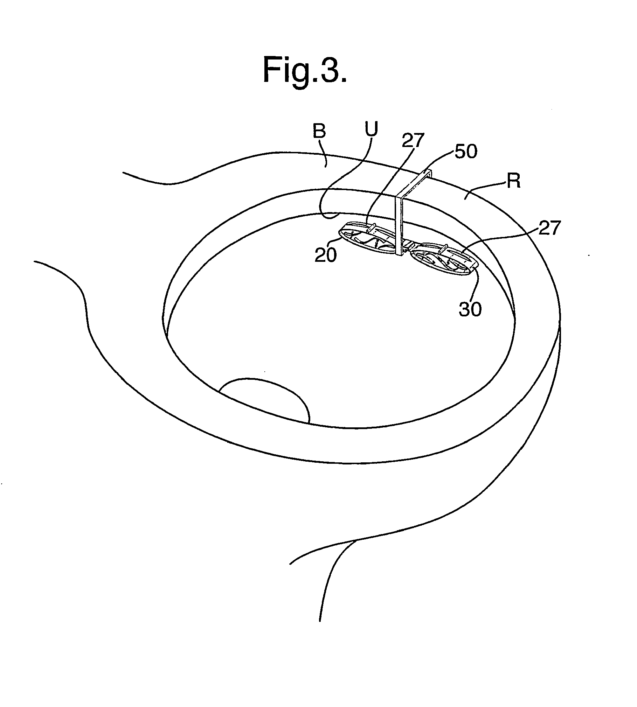

As is visible from Figure 1, the first and second dispenser bodies depend from an intermediate bridge element 40. The relative positioning of the bridge element may vary and is not consequential to the operation of the inventive device and indeed the intermediate bridge element may be comprised of two or more parts which may be joined together to form the bridge element. Desirably however the bridge element is flexibly joined to each respective first and second dispenser bodies such that upon mounting of the device within a sanitary appliance the device may be adapted, e.g., flexed, to a nonlinear configuration as is generally shown with respect to Figure 3 which will be discussed shortly. Adaptive mounting of the device is advantageous such to ensure that sufficient water during a flush cycle enters the body cavity of each of the first and second dispenser bodies of the device.

As is also visible from Figure 1, shown in "phantom" is a representative hanger 50 which depends at one end from the bridge element. A proximal end of the hanger is affixed within a receptacle 32 which is formed as part of the bridge element, while the distal end 34 is hook shaped and is intended to be adapted to conform to the geometry of at least a part of a sanitary appliance, preferably to a toilet bowl rim. It is to be understood that the hanger need not depend from the bridge element, but may depend from other elements or parts of the device. It is also to be understood that a plurality of hangers may also be used.

Not visible on Figure 1 is a device outlet, which may be one or more perforations passing through the exterior sidewall at or near the lowest portions thereof which permits for the egress of water or treatment composition from the device to exit into the sanitary applicance.

Figure 2 illustrates a perspective rear view of the device of Fig. 1. According to this preferred embodiment the body cavity (not visible) is sealed by a cover 36 which facilitates the retention of a chemical composition, especially in the form of a gel and most especially when in the form of a block, within the body cavity.

It is to be understood that while a solid cover is depicted, such is not a requirement and indeed one or more perforations 38 (depicted in phantom) may be present in the cover. The inclusion of such perforations facilitates the egress of water or treatment composition from each of the body cavities of the device after water has entered a body cavity via a body cavity perforation.

Figure 3 illustrates the mounting of the device of Figures 1 and 2 with respect to a toilet bowl "B". As may be seen the hanger 50 is suspended upon a portion of the toilet bowl rim "R" such that the first dispenser body 20 and second dispenser body 30 is positioned to be at least partially beneath the underside "U" of the toilet bowl rim, such that the body cavity perforations 27 are positioned within the flow path of water when the toilet is flushed. The hanger element is desirably sufficiently flexible so that the device may be adapted to the curved sidewall geometry of the interior of the toilet bowl.

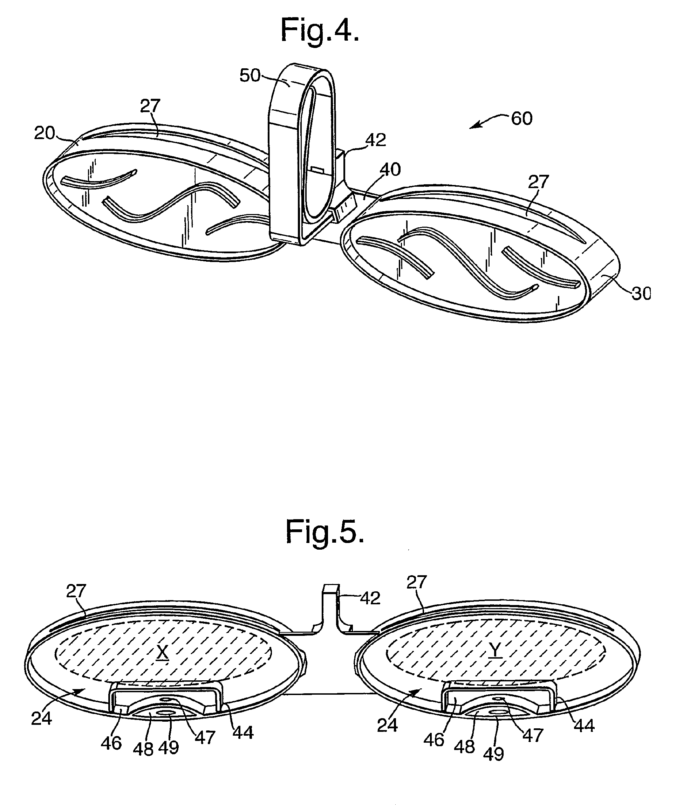

Figure 4 illustrates a perspective frontal view of a second preferred embodiment of a dispensing device 60 according to the invention. In the illustrated embodiment each of the first dispenser body 20 and second dispenser body 30 depends from a bridge element 40 and each includes a single body cavity perforation 27. An upwardly directed buttress wall 42 depending from the bridge element is visible; although optional its inclusion provides a stand-off between the body cavity perforations and the sanitary appliance and decreases the likelihood of blockage of either body cavity perforation from the inlet of flowing water.

Figure 5 illustrates a partial perspective rear view of the device of Fig. 4, illustrating the interior detail of each body cavity, absent the cover which is to be understood to be otherwise present. Within the interior of each body cavity 24 is visible a representative chemical composition "X" and "Y" in the form of a solid block each block resting on a support shelf 44. Each support shelf has a width dimension which is slightly smaller than the interior width of the body cavity which ensures that water flowing into the body cavity via a body cavity perforation contacts a chemical composition (when present) thereby forming a treatment composition and subsequently exiting a respective dispenser body. In the depicted embodiment, the water or treatment composition may pass about the support shelf into a first chamber 46, then through an outlet 47 into a second chamber 48 and which provides for fluid communication therebetween, and ultimately through a device outlet 49 where it exits the device.

It will be realized then that water entering each body cavity necessarily contacts the chemical composition present and form a treatment composition, before exiting the body cavity past the support shelf 44 and enter the first chamber, and thereafter via the outlet 47, next enter the second chamber 48 before ultimately exiting the device via the device outlet 49. In many cases the amount or residence time is a key to ensuring that effective concentrations of a chemical composition are combined with the flush water passing through the device and thus forming the treatment composition. Such may be particularly important wherein the amount of a sanitizing agent such as a bleach or a quaternary ammonium compound is to be eluted and provided to the toilet bowl. Increasing the residence time in the device typically also increases the ultimate concentration of a treatment agent in the treatment composition provided to the toilet bowl. Careful selection of the relative dimensions of the first and second chambers and particularly their respective volumes, as well as for the size and number of outlet opening(s) between the first and second chambers, as well as the size and number of device outlet opening(s) will permit for the production of a device which has a long residence time of the flush water contacting the chemical composition, as well as a longer or delayed delivery period of the delivery of the treatment composition to the toilet bowl following the flush of the toilet bowl. The various dimensions of the first and second chambers can be varied to adjust the residence time of the flush water in each of the first and second dispenser bodies and/or to adjust the rate of delivery of the treatment composition. Additionally or alternately the residence time of the flush water in each of the first and second dispenser bodies can be increased by reducing the cross-sectional area or diameter of the outlet 47. The rate of delivery of the treatment composition to the toilet bowl or other sanitary appliance can also be slowed or delayed by reducing the cross-sectional area or diameter of the device outlet 49.

Further, increasing and/or decreasing the volumes of the first chamber, and of the second chamber may also be used to vary the flowrate of the treatment composition exiting the device. For example wherein a long delay may be desired after the flush of water in the toilet, but prior to release of treatment composition into the toilet bowl, the respective volumes of the of the first chamber, and the second chamber may be reduced, such as to about 20-5:1. The larger volume of the second chamber permits for a longer residence time of the treatment composition in the second chamber.

It is to be understood that although the embodiment of the device according to Figures 1 - 3 include only a device outlet in the form of one or more perforations, it is nonetheless to be understood that the interior elements of the body cavity described with reference to Fig. 5 may be included in the construction of the device according to Figures1-3, 6-8, and 13-19.

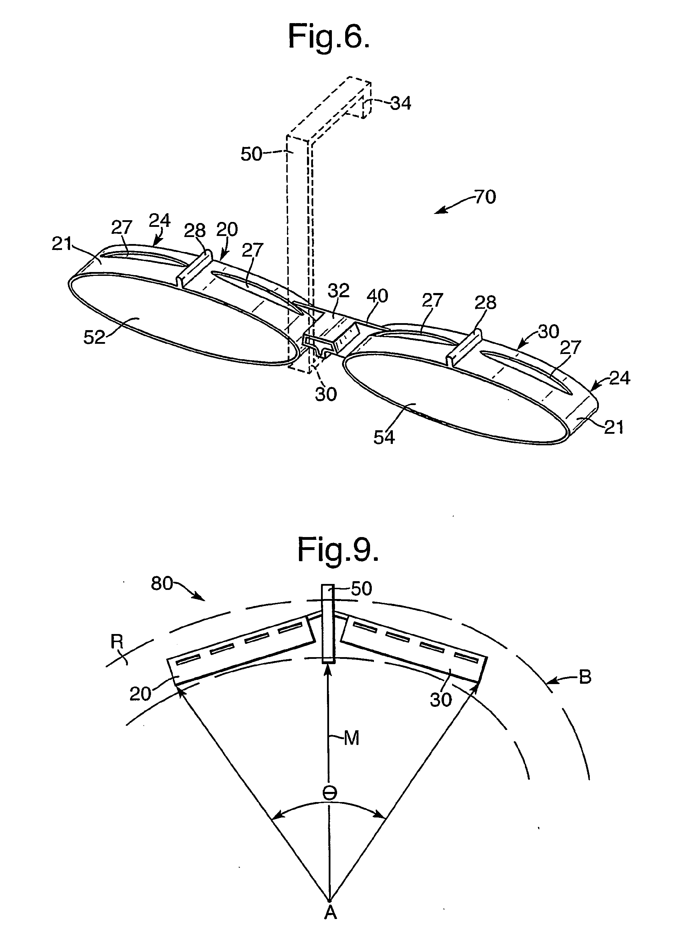

Figure 6 illustrates a perspective frontal view of a dispensing device 70, which is similar in many respects to the device depicted on Figs. 1 - 3, however the device 70 omits a fragrance cavity and divider wall, but includes an two end walls 52, 54 which define the bottom of each body cavity 24 of the respective first dispenser body 20 and second dispenser body 30.

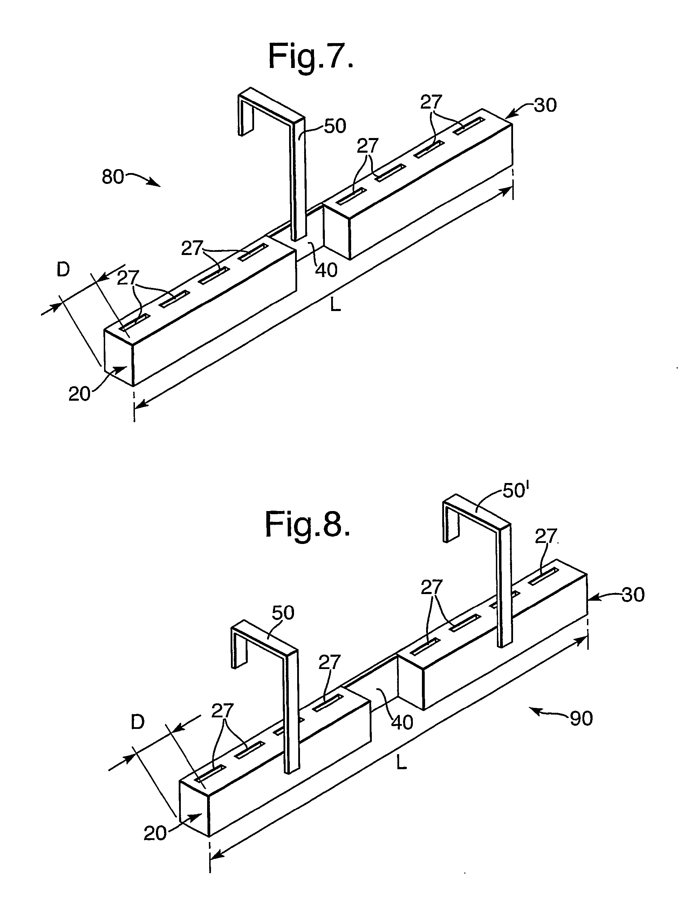

Figure 7 illustrates a perspective frontal view of an alternative embodiment 80 of a dispensing device. The device 80 includes a first dispenser body 20 and second dispenser body 30, each depending from an intermediate bridge element 40. A representative hanger 50 used for suspending the device 80, e.g, upon a portion of a toilet bowl also depends from the intermediate bridge element 40. Each of the first dispenser body 20 and second dispenser body 30 include body cavity perforations 27 each of which define a passage into the body cavity where a chemical composition is present. While not visible on Figure 7, each of the first dispenser body 20 and second dispenser body 30 include a device outlet, which may be one or more perforations passing through the exterior sidewall at or near the lowest portions thereof which permits for the egress of water or treatment composition from the device to exit into the sanitary appliance.

The embodiment illustrated on Fig. 7 depicts a device which has an overall length, indicated by "L" which is at least 5 times, preferably at least 7 times, especially preferably at least 9 times and most preferably at least 10 the maximum dimension "D" of the cross-section of either the first dispenser body 20 and/or the second dispenser body 30, which maximum direction in the depicted embodiment is the diagonal of the cross-section. Such measurements are in accordance with the device 80 is laid upon a flat surface and flattened out to fully extend the device to its maximum length, e.g, as depicted on Figs. 7 and 8. In accordance with such an embodiment, the device provides a slim profile which provides the dual benefits of both reduced visibility when installed within a sanitary appliance, such as under the rim "R" of a toilet bowl "B" as depicted on Figure 9, as well as extending over a wider region of the sidewall of the toilet bowl. While the former benefit is primarily one of an attractiveness and consumer acceptance standpoint, the latter benefit is an important technical benefit which is not believed to have been available in prior art lavatory cleaning devices intended to be used as ITB devices. Placement over a wider region of the sidewall provides more effective cleaning due to better distribution of the treatment composition to the water being flushed around the sidewalls during the flush cycle. According to certain particularly preferred embodiments, the length "L" is at least 5 inches, preferably at least 6 inches and most preferably at least 6.5 inches in length.

Figure 7 all illustrates that the first dispenser body 20 also includes an endpoint "E" at the end of the first dispenser body 20, and a second endpoint "F" which while not shown is to be understood to be at the end of the second dispenser body 30. It is to be understood that the distance between the endpoints "E" and "F" define the distance "L", and such endpoints "E" and "F" are present in all of the illustrated embodiments although not necessarily shown in each of the figures.

Figure 8 illustrates a perspective frontal view of a dispensing device 90 which is in many respects similar to the embodiment illustrated on Fig. 7. The present embodiment however includes a pair of hangers, a first hanger 50 dependent from the first dispenser body 20, and a second hanger 50' dependent from the second dispenser body 30.

Figure 9 illustrates a top planar view of the embodiment of the device 80 disclosed on Figure 7, mounted on a portion of the rim R a toilet bowl B. A portion of the hanger 50 rests atop the rim R, suspending the first dispenser body 20 and second dispenser body 30 beneath the rim. Figure 9 Illustrated are also the respective endpoints E, F which a collinear with the midpoint "M" of the device body dispenser body which is on the midpoint of a plane passing through the dispenser body 20 perpendicular to the endpoints E, F at a distance "x" midway the maximum overall length dimension "L" of the dispenser body 20. The angle of deflection "a" of the midpoint of at least one end of the dispenser body, as measured between an original, of undeformed linear configuration as illustrated in Fig. 7, is and its deformed configuration as measured from the midpoint between the two ends of the dispenser body is at least 5°.

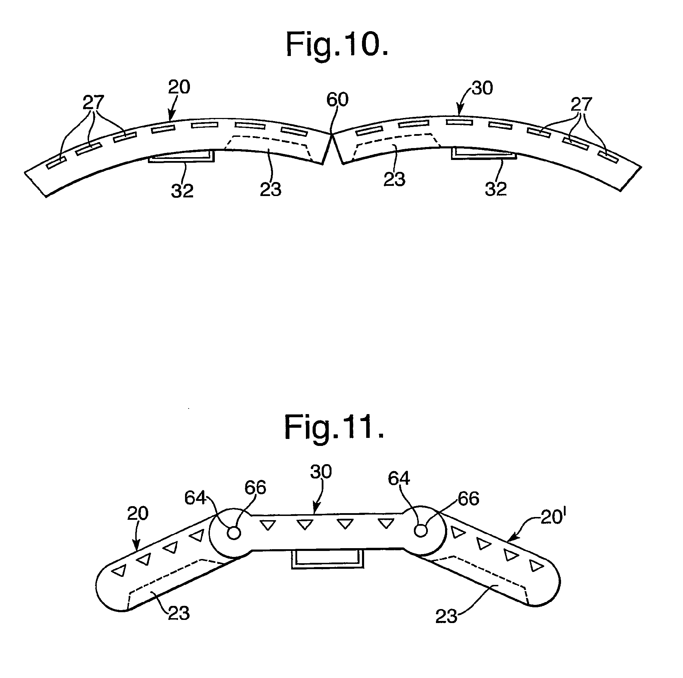

Turning now to Figure 10 there is depicted a dispensing device which comprises a first dispenser body 20 and the second dispenser body 30 flexibly depend from a hinge 60 in the absence of a bridge element. As depicted, the hinge 60 is integrally formed and links one edge or part of the first dispenser body 20 with one edge or part of the second dispenser body 30, permitting the first dispenser body 20 and the second dispenser body 30 to move with respect to one another. Each of the first dispenser body 20 and the second dispenser body 30 further includes a plurality of body cavity perforations 27 which define passages through the body sidewall from the upper exterior sidewall and into the body cavity, as well as a receptacle 32 adapted to receive one end of a hanger (not shown) which may be used to suspend the device within the interior of a sanitary appliance. The embodiment also illustrates, as dotted lines, a fragrance cavity 23 adapted to contain a quantity of a fragrance composition within each of the first dispenser body 20 and the second dispenser body 30, although it is to be understood that such a fragrance cavity may be omitted from one or more of the dispenser bodies depicted on the Figure.

Figure 11 depicts an embodiment wherein common hinges 64 are used to pivotabally link two first dispenser bodies 20' and 20 " to a second dispenser body 30. As depicted the hinges 64 take the form of knuckle-type hinges which include interlocking parts which are pivotabally flexible about hinge pins 66. The hinges provide for a significant degree of flexibility and in certain embodiment is preferred. As is also visible the embodiment depicted on the figure comprises three dispenser bodies; a first dispenser body 20' hingedly linked to a second dispenser body 30 which in turn is hingedly linked to a further first dispenser body 20". flexibly depend from a common hinge, which may function as a bridge element. The embodiment also illustrates, as dotted lines, a fragrance cavity 23 adapted to contain a quantity of a fragrance composition within each of the first dispenser body 20 and the second dispenser body 30, although it is to be understood that such a fragrance cavity may be omitted from one or more of the dispenser bodies depicted on the Figure.

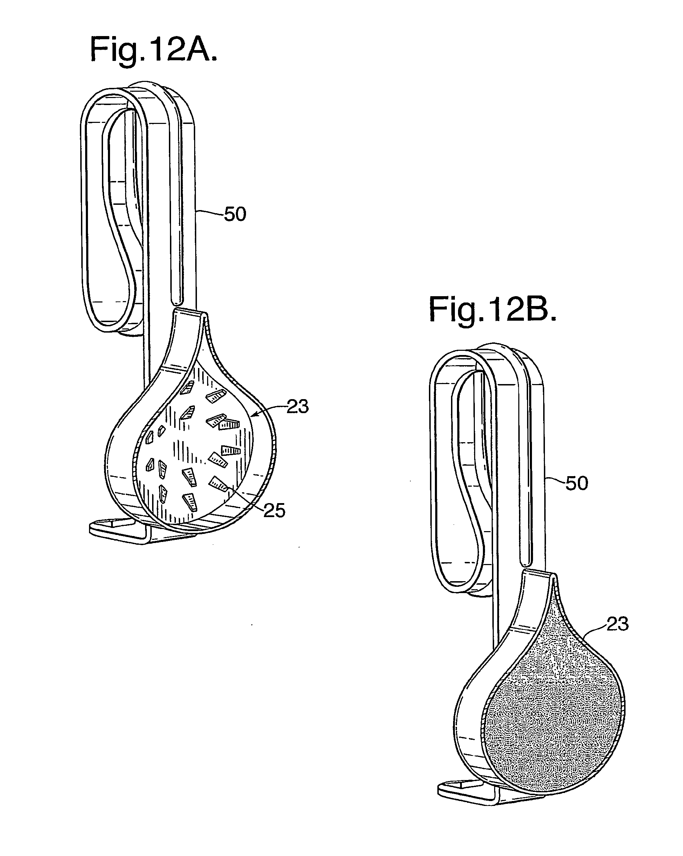

Figures 12A and 12B depict embodiments of a hanger 50 which comprises a fragrance cavity 23 which may be used with any aspect of the invention, including those which may include or which may omit fragrance cavities positioned elsewhere on the device. Figure 12A depicts a hanger 50 which includes depending therefrom, or which may be integrally formed therewith a fragrance cavity 23, here depicted prior to being filled with fragrance composition in order to depict a plurality of short support prongs 25 which are formed to extend into the interior of the fragrance cavity 23 and to provide a retaining means for the gel composition. Figure 12B depicts a hanger 50, and a fragrance cavity 23' which is filled with a fragrance composition.

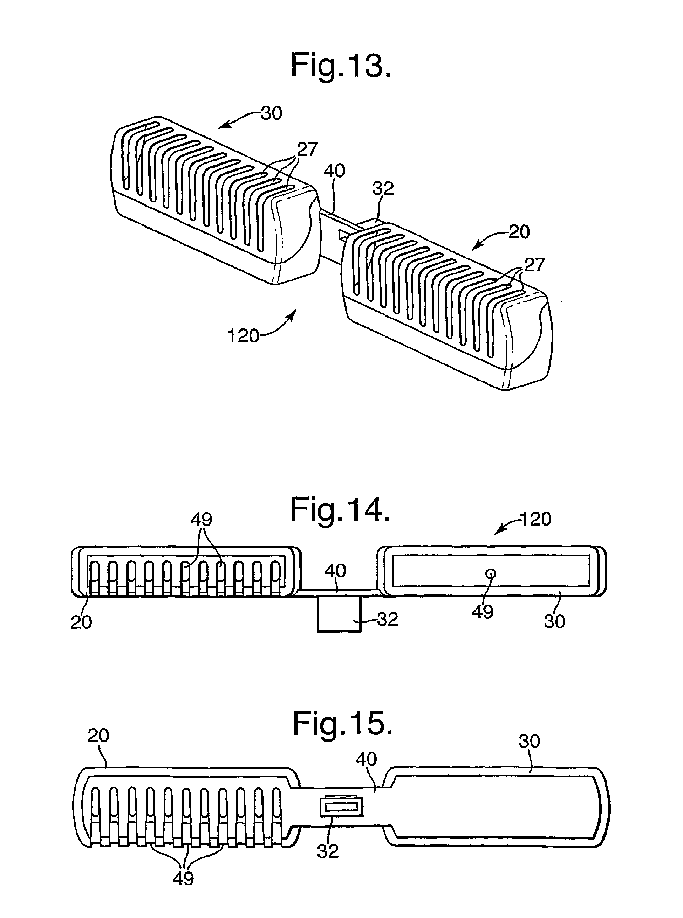

Figure 13 illustrates a perspective frontal view of a further embodiment of a dispensing device 120 which is in many respects similar to the embodiment illustrated on Figure 7. The present embodiment however comprises body cavity perforations 27 which in the embodiment depicted are slots extending over the top and/or down the front of the device and which provide for passages for the inlet of water into the interior of each body cavity. For one body cavity the device outlets 49 take the form of slots on the bottom and/or rear portion of the device, while for the other body cavity as one or more holes as illustrated in Figure 14. It is fully contemplated however that in addition to such slots or holes that other orifices of different geometries or configurations may be used as well. The two dispenser bodies 20 and 30 of the device 120 may have identical or differing body cavity perforations and device outlets. Also depicted is a receptacle 32 which is formed as part of the bridge element, adapted to receive a hook or hanger (not shown).

Figure 14 is a bottom planar view of the embodiment illustrated on Figure 13.

Figure 15 is a rear planar view of the embodiment illustrated on Figure 13.

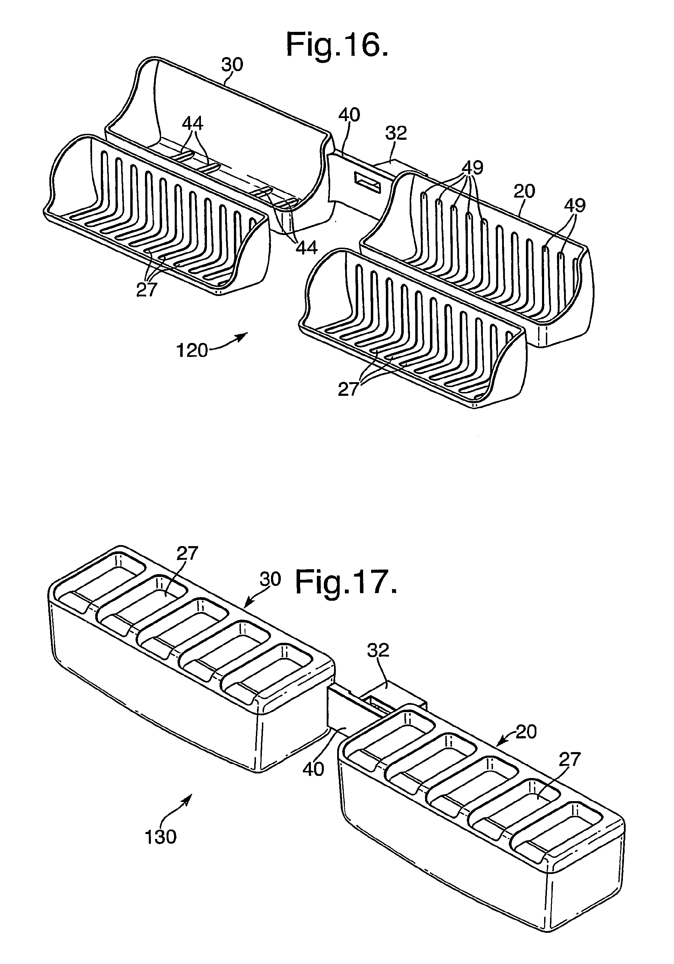

Figure 16 is a perspective frontal view of the embodiment illustrated on Figure 13 with the upper halves of the dispensing bodies separated from the bottom halves to show the internal structure of these bodies.

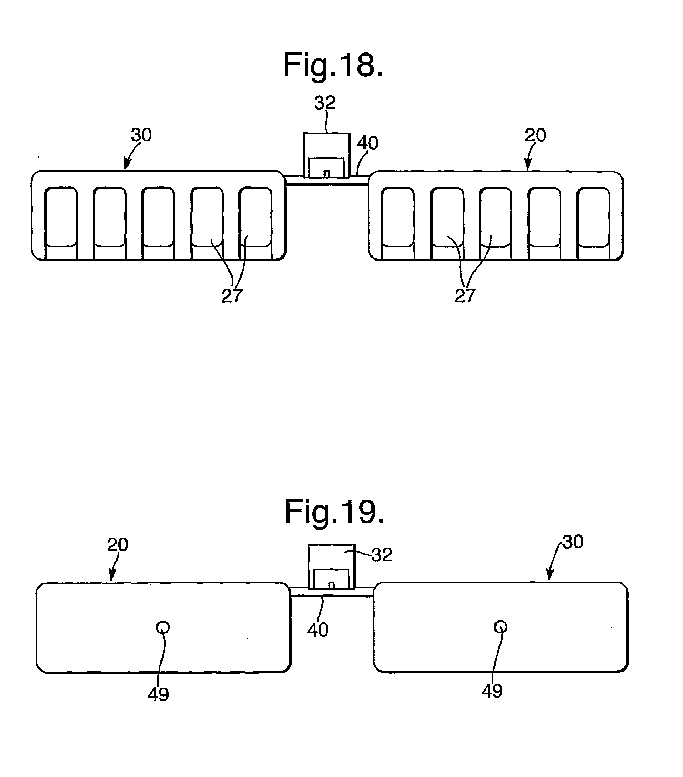

Figure 17 illustrates a perspective frontal view of a further embodiment of a dispensing device 130 according to the second aspect of the invention, which is in many respects similar to the embodiment illustrated on Figure 7. The device outlets 49 may take the form of slots on the bottom and/or rear portion of the device similar to the embodiment illustrated on Fig. 13, or as holes as illustrated in figure 19. The two dispenser bodies 20 and 30 of the device 130 have according to the invention differing body cavity perforations and device outlets. Also depicted is a receptacle 32 which is formed as part of the bridge element, adapted to receive a hook or hanger (not shown).

Figure 18 is a top planar view of the embodiment illustrated on Figure 13.

Figure 19 is a bottom planar view of the embodiment illustrated on Figure 13.

It is to be understood that while the embodiments illustrated on Figs. 7 through 11 depict devices according to an aspect not according to the invention, the first dispenser body 20 and second dispenser body 30 may also be of configuration whereby each includes a fragrance cavity adapted for receiving a fragrance composition as disclosed in one or more of the prior Figures 1- 6.

An important feature of the dispensing devices according to Figs. 13-19 is that the body cavity inlets and outlets on the two different dispenser bodies may be of different shapes or sizes, and thus allow for differing egress rates for the compositions formed by the contact of water with the material retained inside the different dispensing bodies. This is illustrated for example in Fig. 14 where the device outlets on one dispenser body comprise slots, while on the other dispenser body a single hole forms the outlet. Such a configuration provides means whereby treatment compositions formed in the dispensing bodies may be delivered at different rates, e.g. fluid flow rates to a sanitary appliance such as a toilet bowl.

This is a particularly useful feature if one of the dispenser bodies contains a surfactant material while an other contains disinfectant material, most preferably bleach or another germicidal compound. The surfactant material could be placed in the dispenser body that allowed for a quick rate of egress so that the surfactant composition would enter the sanitary device, such as a toilet bowl, while the flush cycle was in progress, and the flushing action would aid in the cleaning of the inside of the bowl with the surfactant composition. The disinfectant material composition could be placed in the dispenser body with the lower rate of egress so that the disinfecting composition continued to be released from the dispenser body even after the flush cycle was completed so that some of this composition would remain in the bowl between uses to provide a continuing disinfecting action.

Additionally, the use of two separate dispenser bodies providing treatment compositions at different flow rates permits the delivery of chemically incompatible treatment compositions to a sanitary appliance.

It is to be understood that references to directions, e.g., frontwardly, rearwardly, and upwardly are included for sake of convenient reference.

### Examples

Blocks having the compositions on the following table were produced by extruding the constituents into blocks having a size of 20mm by 10 mm by 75 mm, which provides a block having surface area/volume ratio of 4500 mm²/15000 mm³, or 0.3.

| Table 1 | | | | | | | | | | | | | |
|---|---|---|---|---|---|---|---|---|---|---|---|---|---|
| | C1 | E1 | E2 | E3 | E4 | E5 | E6 | E7 | E8 | E9 | E10 | E11 | E12 |
| AOS | 32.0 | 27.0 | 27.0 | 15.0 | 15.0 | 15.0 | 15.0 | 23.59 | 20.18 | 15.0 | 27.0 | 27.0 | 27.0 |
| LMEA (98%) | 5.0 | 30.0 | 30.0 | 22.0 | 15.0 | 30.0 | 30.0 | 25.77 | 21.55 | 30.0 | 15.0 | 15.0 | 15.0 |
| DDBS (80%) | 35.0 | 15.0 | 15.0 | 35.0 | 32.0 | 17.0 | 17.0 | 20.05 | 25.09 | 27.0 | 20.0 | 30.0 | 20.0 |
| Na Sulfate | 21.0 | 21.0 | 21.0 | 21.0 | 31.0 | 31.0 | 31.0 | 23.59 | 26.18 | 21.0 | 31.0 | 21.0 | 31.0 |
| Na lauryl ether sulfate (70%) | 5.0 | 5.0 | 5.0 | 5.0 | 5.0 | 5.0 | 5.0 | 5.0 | 5.0 | 5.0 | 5.0 | 5.0 | 5.0 |
| Silica | 2.0 | 2.0 | 2.0 | 2.0 | 2.0 | 2.0 | 2.0 | 2.0 | 2.0 | 2.0 | 2.0 | 2.0 | 2.0 |

The specific identity of the constituents used to produce the blocks are described more fully in the following table:

| Table 2 | |
|---|---|
| Constituent: | Identity: |
| AOS (95-100%) | alpha olefin sulfonate, sodium salt, 95-100%wt. actives |
| LMEA (98%) | lauramide monoethanolamide, 98%wt. actives |
| DDBS (80%) | anionic surfactant, dodecylbenzene sulfonate, 80%wt. actives |
| Na sulfate | sodium sulfate, 100%wt. actives |
| Na lauryl ether sulfate (70%) | anionic surfactant, sodium lauryl ether sulfate, 70%wt. actives |
| Silica | anhydrous silica, 100%wt. actives |

The performance characteristics of the blocks were evaluated by placing them into a dispensing device similar to that according to Fig. 7 and suspending the block and the device beneath the rim of a standard toilet and in the path of flush water released from the cistern. The blocks were weighed initially, and then subsequent to 24, 108, 132, 168, 216, 264 and 336 flush cycles. The weight deviation from the original block weight is reported on the following table.

| Table 3 | | | | | | | |
|---|---|---|---|---|---|---|---|
| | %wt. loss (-) / %wt. gain (+) | | | | | | |
| Flushes: | 24 | 108 | 132 | 168 | 216 | 264 | 336 |
| C1 | -5 | -35 | -48 | -68 | -90 | - | - |
| E1 | +9.7 | +2.7 | -13.6 | -41.2 | -85.1 | - | - |
| E2 | +9.3 | +15.2 | +5 | -12.6 | -53.3 | -65.5 | -80.1 |
| E3 | +9.4 | +1.1 | -9.2 | -19.7 | -56.7 | -74.5 | -88.4 |
| E4 | +5.5 | +3.2 | -5.2 | -13.6 | -32.0 | -54.1 | -69.5 |
| E5 | +7.5 | +19.2 | +16.7 | +13.1 | -3.6 | -34.2 | -87.1 |
| E7 | +5.6 | -7.3 | -22.2 | -40.6 | -67.1 | -77 | -94.2 |
| E8 | +5.3 | -9.6 | -25.9 | -50.3 | -94.2 | - | - |
| E9 | +10.5 | +16.3 | +7.7 | -0.1 | -21.6 | -37.5 | -63.9 |
| E10 | -2.2 | -31.2 | -47.3 | -62.0 | -81.4 | -85.3 | - |
| E12 | +2.7 | -41.7 | -61.0 | -76.3 | -89.0 | - | - |

As is evident from the foregoing table, certain of the block were noted to initially gain weight which is attributed to water absorption, and indicated by positive "+" percentages. Weight losses are indicated by negative "-" percentages, and where no composition was remaining in the device, such is noted by "-" in the table, signifying that the block was consumed prior to the flush cycle indicated.

The blocks were also evaluated for their foaming characteristics following use. Following the 24^{th} flush cycle of the foregoing test, the appearance of the foam within the interior of the toilet bowl was visually observed and evaluated, then scored. The results of this evaluation are indicated on the following table, wherein the scores were as follows:
"4" = a thick foam layer covering the complete surface of the water in the bowl;
"3" = a thinner foam layer covering the complete surface of the water in the bowl;
"2" = a foam layer incompletely covering the surface of the water in the bowl;
"1" = visible foam at the peripheral edges of the water in the bowl and the sidewall of the toilet bowl;
"0" = no visible foam on the surface of the water in the bowl.
The results of this evaluation are reported on the following table.

| Table 4 | |
|---|---|
| | Foam Rating following 24 flushes |
| E1 | 3 |
| E2 | 2 |
| E3 | 3 |
| E4 | 3 |
| E5 | 2 |
| E7 | 3 |
| E8 | 3 |
| E9 | 3 |
| E10 | 4 |
| E12 | 3 |

As is visible from the foregoing, the blocks provided good foaming performance and long lasting performance in the dispensing devices of the invention.

While the invention is susceptible of various modifications and alternative forms, it is to be understood that specific embodiments thereof have been shown by way of example which are not intended to limit the invention to the particular forms disclosed; on the contrary the intention is to cover all modifications, equivalents and alternatives falling within the scope as expressed in the appended claims.

## Claims

1. A toilet bowl dispensing device which delivers at least one treatment composition selected from: coloring compositions, cleaning compositions, bleaching compositions and disinfecting compositions, anti-limescale compositions to a portion of a toilet bowl, while simultaneously providing a fragrancing effect to the ambient environment of the toilet bowl,
wherein the device includes:
(a) a first dispenser body and a second dispenser body, each comprising a body cavity containing:
• at least one said treatment composition;
• at least one inlet to permit for passage of water contained within the device to pass into the body cavity and contact said treatment composition; and,
• at least one outlet to permit for the egress of a treatment composition from the body cavity;
at least one of each dispenser body comprising a fragrance cavity containing a quantity of a fragrance composition;
(b) a bridge element from which the first dispenser body and the second dispenser body depend; and,
(c) a hanger which depends from the bridge element, which hanger is adapted for removably hanging the device upon a portion of a toilet bowl,
wherein the at least one treatment composition contained in the first dispenser body is different from the at least one treatment composition contained in the second dispenser body; and,
**characterized in that**
the body cavity inlets and outlets on the two different dispenser bodies are of different shapes or sizes to provide different egress rates for the treatment compositions exiting the body cavities.

2. A dispensing device according to claim 1 wherein the first dispenser body contains a fragrance in the fragrance cavity, and at least one chemical composition in the body cavity, and further wherein the second dispenser body also contains a fragrance in the fragrance cavity, and at least one chemical composition in the body cavity.

3. A dispensing device according to claims 1 or 2 wherein the fragrance compositions of the first dispenser body and of the second dispenser body are the same.

4. A dispensing device according to claims 1 or 2 wherein the fragrance compositions of the first dispenser body and of the second dispenser body are different.

5. A dispensing device according to any preceding claim wherein the first dispenser body and the second dispenser body flexibly depend from the bridge element.

6. A dispensing device according to any preceding claim which comprises three or more dispenser bodies, at least two dispenser bodies of which depend from a bridge.

7. A dispensing device according to any preceding claim wherein the hanger comprises a fragrance cavity.

8. A device according to any preceding claim wherein the treatment composition is in the form of a solid block.

9. A dispensing device according to claim 8 whererin the dispensing device contains a cleaning block which comprises:
10 - 35%wt., of an alpha olefin sulfonate anionic surfactant;
10 - 35%wt., of a linear monoethanolamide;
5 - 50 %wt., of a linear dodecylbenzene sulfonate anionic surfactant;
5 - 50%wt., of sodium sulfate
0.1 - 15 %wt., of silica
0.1 - 25%wt., of sodium lauryl ether sulfate
optionally to 40%wt. further additive constituents, including but not limited to further surfactants, fillers, binders, fragrances, processing aids such as lubricants and tabletting aids, bleaches, stanitizing compositions.

10. A device according to any of claims 1- 8 wherein the treatment composition is in the form of a gel.

11. A process for delivering a plurality of treatment compositions to the interior of a toilet bowl, whereby at least one treatment composition is delivered at a different rate as compared to at least one other treatment compound, which process uses a device as described with reference to any of claims 1-10, installing said device within or upon at least a portion of a toilet bowl, whereby said treatment composition contacts water contained within the toilet bowl.

## Patentansprüche

1. Spendervorrichtung für eine Toilettenschüssel, die einem Teil einer Toilettenschüssel wenigstens eine aus Färbezusammensetzungen, Reinigungszusammensetzungen, Bleichzusammensetzungen und Desinfektionszusammensetzungen, Anti-Kalk-Zusammensetzungen ausgewählte Behandlungszusammensetzung zuführt und dabei gleichzeitig eine Beduftungswirkung für die Umgebung der Toilettenschüssel bereitstellt,
wobei die Vorrichtung aufweist:
(a) einen ersten Spenderkörper und einen zweiten Spenderkörper, die jeweils einen Körperhohlraum umfassen, welcher
• wenigstens eine der Behandlungszusammensetzungen;
• wenigstens einen Einlass, um ein Hindurchgelangen von in der Vorrichtung enthaltenem Wasser zu erlauben, so dass es in den Körperhohlraum gelangt und die Behandlungszusammensetzung berührt; und
• wenigstens einen Auslass, um das Austreten einer Behandlungszusammensetzung aus dem Körperhohlraum zu erlauben,
enthält;
wobei wenigstens einer der Spenderkörper einen Duftstoffhohlraum umfasst, der eine Menge einer Duftstoffzusammensetzung enthält;
(b) ein Brückenelement, von dem der erste Spenderkörper und der zweite Spenderkörper abhängen; und
(c) einen Aufhänger, der von dem Brückenelement abhängt, wobei der Aufhänger dazu geeignet ist, die Vorrichtung entfernbar an einem Teil einer Toilettenschüssel aufzuhängen,
wobei die wenigstens eine in dem ersten Spenderkörper enthaltene Behandlungszusammensetzung sich von der wenigstens einen in dem zweiten Spenderkörper enthaltenen Behandlungszusammensetzung unterscheidet; und
**dadurch gekennzeichnet, dass**
die Körperhohlraumeinlässe und -auslässe an den zwei unterschiedlichen Spenderkörpern unterschiedliche Formen oder Größen haben, um unterschiedliche Austrittsraten für die aus den Körperhohlräumen austretenden Behandlungszusammensetzungen bereitzustellen.

2. Spendervorrichtung gemäß Anspruch 1, wobei der erste Spenderkörper in dem Duftstoffhohlraum einen Duftstoff und in dem Körperhohlraum wenigstens eine chemische Zusammensetzung enthält, und wobei weiterhin der zweite Spenderkörper ebenfalls in dem Duftstoffhohlraum einen Duftstoff und in dem Körperhohlraum wenigstens eine chemische Zusammensetzung enthält.

3. Spendervorrichtung gemäß Anspruch 1 oder 2, wobei die Duftstoffzusammensetzungen des ersten Spenderkörpers und des zweiten Spenderkörpers dieselben sind.

4. Spendervorrichtung gemäß Anspruch 1 oder 2, wobei die Duftstoffzusammensetzungen des ersten Spenderkörpers und des zweiten Spenderkörpers unterschiedlich sind.

5. Spendervorrichtung gemäß einem der vorhergehenden Ansprüche, wobei der erste Spenderkörper und der zweite Spenderkörper flexibel von dem Brückenelement abhängen.

6. Spendervorrichtung gemäß einem der vorhergehenden Ansprüche, die drei oder mehr Spenderkörper umfasst, von denen wenigstens zwei Spenderkörper von einer Brücke abhängen.

7. Spendervorrichtung gemäß einem der vorhergehenden Ansprüche, wobei der Aufhänger einen Duftstoffhohlraum umfasst.

8. Vorrichtung gemäß einem der vorhergehenden Ansprüche, wobei die Behandlungszusammensetzung die Form eines festen Blocks hat.

9. Spendervorrichtung gemäß Anspruch 8, wobei die Spendervorrichtung einen Reinigungsblock enthält, welcher umfasst:
10 - 35 Gew.-% eines anionischen Alpha-Olefinsulfonat-Tensids;
10 - 35 Gew.-% eines linearen Monoethanolamids;
5 - 50 Gew.-% eines anionischen linearen Dodecylbenzolsulfonat-Tensids;
5 - 50 Gew.% Natriumsulfat;
0,1 - 15 Gew.-% Silica;
0,1 - 25 Gew.-% Natriumlaurylethersulfat;
optional bis zu 40 Gew.% weitere Zusatzbestandteile, darunter, aber nicht ausschließlich, weitere Tenside, Füllstoffe, Bindemittel, Duftstoffe, Verarbeitungshilfsstoffe wie Schmierstoffe und Tablettierhilfen, Bleichmittel, hygienisierende Zusammensetzungen.

10. Vorrichtung gemäß einem der Ansprüche 1 - 8, wobei die Behandlungszusammensetzung die Form eines Gels hat.

11. Verfahren zum Zuführen einer Vielzahl von Behandlungszusammensetzungen in das Innere einer Toilettenschüssel, wobei wenigstens eine Behandlungszusammensetzung im Verhältnis zu wenigstens einer anderen Behandlungszusammensetzung mit einer unterschiedlichen Rate abgegeben wird, wobei das Verfahren eine Vorrichtung wie die bezüglich eines der Ansprüche 1 - 10 beschriebene verwendet und die Vorrichtung in oder an wenigstens einem Teil einer Toilettenschüssel installiert wird, wobei die Behandlungszusammensetzung in der Toilettenschüssel enthaltenes Wasser berührt.

## Revendications

1. Un dispositif de distribution pour cuvette de toilette qui délivre au moins une composition de traitement sélectionnée parmi: compositions de coloration, compositions d'entretien, compositions de blanchiment et compositions de désinfection, composition anti calcaire à une partie d'une cuvette de toilette, tout en fournissant simultanément un effet parfumant à l'environnement ambiant de la cuvette de toilette,
où le dispositif comprend
(a) un premier corps de distributeur et un second corps de distributeur, chacun comprenant une cavité de corps contenant:
• au moins une dite composition de traitement
• au moins une entrée pour permettre un passage d'eau contenu dans le dispositif de sorte à passer dans la cavité de corps et entrer en contact avec ladite composition de traitement; et
• au moins une sortie pour permettre l'échappement d'une composition de traitement de la cavité de corps:
au moins un de chacun des corps de distribution comprenant une cavité de parfum contenant une quantité d'une composition de parfum
(b) un élément pont depuis lequel le premier corps de distribution et le second corps de distribution dépendent, et
(c) un crochet qui dépend de l'élément pont, lequel crochet est adapté pour accrocher le dispositif de manière mobile sur une portion de cuvette de toilette,
où l'au moins une composition de traitement contenue dans le premier cors de distribution est différente de l'au moins une composition de traitement contenue dans le second corps de distribution; et
**caractérisé en ce que**
les entrées et sorties de cavités de corps sur les deux corps de distribution différents sont de formes ou tailles différentes pour fournir différent taux d'échappement pour les compositions de traitement quittant les cavités de corps.

2. Un dispositif de distribution selon la revendication 1 où le premier corps de distribution contient un parfum dans la cavité de parfum et au moins une composition chimique dans la cavité de corps et de plus où le second corps de distribution contient aussi un parfum dans la cavité de parfum et au moins une composition chimique dans la cavité de corps.

3. Un dispositif de distribution selon les revendications 1 ou 2 où les compositions de parfum du premier corps de distribution et du second corps de distribution sont les mêmes.

4. Un dispositif de distribution selon les revendications 1 ou 2 où les compositions de parfum du premier corps de distribution et du second corps de distribution sont différentes.

5. Un dispositif de distribution selon l'une des revendications précédentes où le premier corps de distribution et le second corps de distribution dépendent de manière flexible de l'élément pont.

6. Un dispositif de distribution selon l'une des revendications précédentes qui comprend trois ou plus de corps de distribution, au moins deux corps de distribution dépendent d'un pont.

7. Un dispositif de distribution selon l'une des revendications précédentes où le crochet comprend une cavité de parfum.

8. Un dispositif de distribution selon l'une des revendications précédentes où la composition de traitement est dans la forme d'un bloc solide.

9. Un dispositif de distribution selon la revendication 8 où le dispositif de distribution contient un bloc d'entretien qui comprend:
10-35% pds d'un surfactant anionique de sulfonate d'alpha-oléfine
10-35% pds d'un monoéthanolamide linéaire
5-50% pds d'un surfactant anionique de dodecylbenzensulfonate linéaire
5-50% pds de sulfate de sodium
0,1-15% pds de silice
0,1-25% pds de sulfate laurique d'éther de sodium
en option jusqu'à 40% pds de constituants additifs supplémentaires incluant mais ne se limitant pas à des surfactants, des matériaux de remplissage, des liants, des parfums, des additifs de traitement comme des lubrifiants et des adjuvants de pastillage, des javellisants, des compositions désinfectantes.

10. Un dispositif de distribution selon l'une des revendications 1 -8 où la composition de traitement est dans la forme d'un gel.

11. Un processus pour délivrer une pluralité de composition de traitement à l'intérieur d'une cuvette de toilette où au moins une composition de traitement est délivrée à un taux différent comparé à au moins un autre composant de traitement, lequel processus utilise un dispositif comme décrit en référence à l'une des revendications 1-10, installer ledit dispositif dans ou sur au moins une portion d'une cuvette de toilette où ladite composition de traitement entre en contact avec l'eau dans la cuvette de toilette.
